# EUROPEAN PATENT APPLICATION

(11) **EP 1 346 734 A1**
(43) Date of publication of application: **24.09.2003**
(21) Application number: 02019164.9
(22) Date of filing: 30.08.2002
(51) Int. Cl.: A61L 9/12, A01M 1/20, A44C 15/00, A45D 34/00

(54) **Device for diffusing a volatile active substance into the environment**

(30) Priority: 11.03.2002 IT MI20020136 U
(71) Applicant: Deoflor S.p.A., 27030 Confienza (Pavia) (IT)
(72) Inventor: Guenzi, Gian Luca, 27038 Robbio (Pavia) (IT); Ruffina, Laura, 27036 Mortara (Pavia) (IT)
(74) Representative: Modiano, Guido, Dr.-Ing.

(57) **Abstract**

A device for diffusing a volatile active substance into the environment, comprising a container (2) that forms a compartment (3) for containing a solid or semisolid product that contains the active substance. The compartment (3) is delimited toward the outside of the container (2) by at least one wall (5) made of transparent and/or semitransparent material. The product (4) is visible through the wall (5) of the container (2) and is transparent and/or semitransparent and/or colored. A decorative element (6) that is visible from the outside of the container (2) is provided on the wall of the container and/or on a part of the product (4) that is visible through the wall (5) of the container (2). The combination of the optical characteristics of the product (4) with those of the decorative element and of the container (2) produces light reflection, refraction and diffusion effects that combine with each other, providing a particular visual effect.

## Description

The present invention relates to a device for diffusing a volatile active substance into the environment, particularly for diffusing a deodorizer and/or fragrance but also usable to diffuse other substances, such as for example insecticides, sanitizers or other substances.

Various kinds of deodorizing and/or fragrance-dispensing products for environments are known.

In particular, liquid products with spray or aerosol dispensing are known which are packaged under pressure in containers of the metal spray-can type; said containers do not have ornamental functions per se but are merely intended to contain the product and to present it, by means of a suitable label, to the potential buyer.

Products that are likewise liquid but are dispensed by natural evaporation through substrates or membranes made of permeable natural or synthetic material, or by an electric heating device, or by micronized spraying without gas under pressure, are also known. These products are generally packaged in containers, made of opaque or clear synthetic material or glass, which have different capacities and shapes. In this case also, although the container is not constrained by the need to withstand the pressure of the product and therefore has fewer shape constraints, the container simply performs the function of containing the product and of presenting it to the potential buyer.

Another type of product is constituted by solid deodorizers and/or fragrances, which can be in the form of gels, resins, polymers, impregnated inert materials, silicas, or others. These products are generally placed in containers, usually made of synthetic material but also made of other materials, which are hollow internally in order to accommodate the deodorizer and/or fragrance and are usually provided with suitable openings to allow the evaporation and dispersion into the environment of the deodorizer and/or fragrance. The solid product can also be placed between two parts with a grille for the evaporation of the active substance. Since these containers must remain exposed for a long time in an environment, in some cases attention is paid to their aesthetic aspects. However, the aesthetic effects are obtained simply by seeking a particular shape of the enclosure, which usually leads to modest results.

International patent application WO-00/24434 discloses a container for deodorizers and/or fragrances that can be made of various materials, particularly transparent material, and has on one of its faces shaped recesses for containing the deodorizer and/or fragrance in the solid or semisolid state and to modulate its evaporation. In this type of container, the product cooperates with the container in obtaining an aesthetic effect that is nonetheless rather limited despite being better than that of conventional containers.

The aim of the present invention is to provide a device for diffusing a volatile active substance into the environment, in which the container and the product that contains the active substance also cooperate in giving the device, as a whole, a functional and also an aesthetic appearance that is distinctly better than that achievable by currently commercially available devices, making the device a true decorative object.

Within the scope of this aim, an object of the invention is to provide a device that ensures correct functionality in the dispersion of the active substance in the environment.

This aim and these and other objects that will become better apparent hereinafter are achieved by a device for diffusing a volatile active substance into the environment, comprising a container which forms a compartment for containing a solid or semisolid product that contains the active substance, characterized in that said compartment is delimited toward the outside of the container by at least one wall made of transparent and/or semitransparent material, said product being visible through said wall of the container and being transparent and/or semitransparent and/or colored, a decorative element that is visible from the outside of said container being provided on said wall of the container and/or on a part of said product that is visible through said wall of the container.

Further characteristics and advantages will become better apparent from the detailed description of the device according to the invention, illustrated only by way of non-limitative example in the accompanying drawings, wherein:
Figure 1 is a front elevation view of the device according to the invention;
Figure 2 is a sectional view of Figure 1, taken along the line II-II;
Figure 3 is a side elevation view of the device according to the invention;
Figure 4 is a sectional view, taken like Figure 2, of a different embodiment of the device according to the present invention.

With reference to the figures, the device according to the invention, generally designated by the reference numeral 1, comprises a container 2 in which there is a compartment or enclosure 3 that contains a product 4 in the solid or semisolid state, which contains the active substance to be diffused.

According to the invention, the compartment 3 is delimited, toward the outside of the container 2, by at least one wall 5, which is made of transparent and/or semitransparent material.

The product 4 is visible through the wall 5 and can be transparent and/or semitransparent and/or colored.

A decorative element 6, 6a, visible from the outside of the container 2, is provided on the wall 5, i.e., on at least one of its two opposite faces and/or inside it, and/or on a part of the product 4 that is visible through the wall 5.

According to the requirements and the effects sought, the product 4 can optionally have multiple colors.

Preferably, the container 2 is formed monolithically by using optionally colored clear or semitransparent glass or synthetic material, and the thickness of the wall 5 is preferably is substantially between 0.3 and 20 mm.

The compartment 3 is open on its opposite side with respect to the wall 5, so as to allow the gradual evaporation and dispersion of the product 4 into the environment in which the device is placed.

Preferably, the refractive index of the product 4 is different from the refractive index of the wall 5 of the container 2.

The product 4 is preferably constituted by a carrier that contains the active substance, which is preferably constituted by a deodorizer and/or a fragrance, to be diffused into the environment.

The carrier can be chosen among the materials usually used to retain fragrances and/or deodorizers or insect repellents or sanitizers. In particular, it is possible to use as carriers for example the following: waxes (paraffin), alkaline salts of fatty acids (stearate), silicas, alginates, carrageenans, synthetic polymers, elastomers, water-based polymers (hydrogel polymers), anhydrous-base polymers (anhydrous polymergels), plastics, silicas, carboxymethylcellulose, cellulose derivatives, etcetera.

The carrier is preferably constituted by a wax or gel and is poured into the compartment 3 so as to adhere to the walls that delimit said compartment 3, in particular so as to adhere to the inner side of the wall 5.

The active substance is present in the product 4 in a percentage, with respect to the total weight of the product 4 (constituted by the carrier and by the active substance), that is comprised between 5 and 90%, preferably between 30 and 80%.

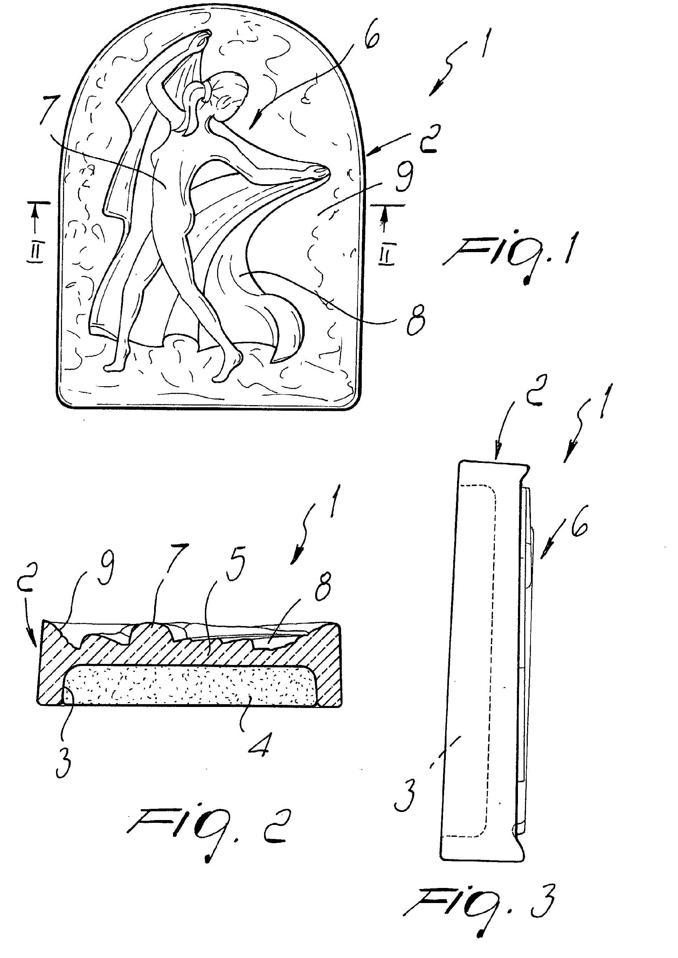

The decorative element 6, which in the embodiment shown in Figures 1 to 3 is constituted by a female figure 7 holding a veil 8, has protruding parts and recessed parts.

In particular, with reference to the example shown in said figures, the female figure 7 is formed in bas-relief on the outer face of the wall 5 and has a smooth surface. The veil 8 has cusps with incisions and reliefs, with angles studied so as to obtain particular optical effects.

The background 9 against which the figure 7 stands out preferably has a surface that is irregular and rough, opaque or semi-opaque or semitransparent.

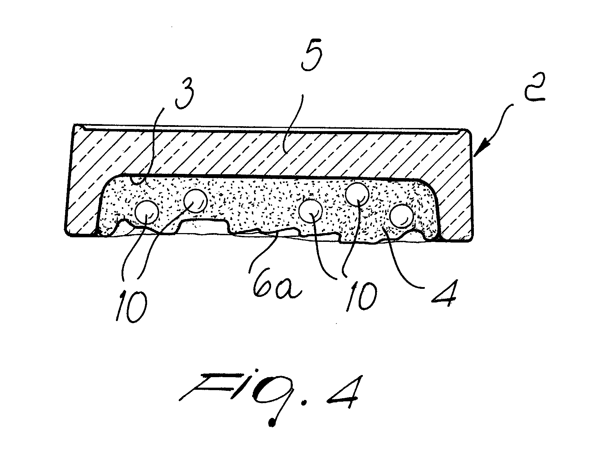

As an alternative or in combination, as shown in Figure 4, the decorative element, designated by the reference numeral 6a in said figure, can be formed in relief and/or recessed, for example by hot or cold molding of the product 4 in solidified form or in the process of solidifying, on the opposite face of the product 4 with respect to the wall 5. It should be noted that the elements of Figure 4 that relate to elements that have already been described with reference to Figures 1 to 3 have been designated by the same reference numerals.

Also as an alternative or in combination, the decorative element can comprise inclusions 10 which have shapes and dimensions that are variable according to the requirements and are embedded in the product 4 and can be visually diversified with respect to said product, for example because they have a different color and/or they are made of a material that has a different refractive index with respect to the product 4 in which they are embedded.

The decorative element may further comprise decorative elements applied to the outer and/or inner face of the wall 5.

The design 6, 6a, as well as the shape of the container 2, may be any according to the requirements.

In practice, the combination of the transparent material of which the container 2 is made and of the coloring and/or transparency of the product 4, as well as the presence of the design 6, 6a in relief and/or recessed on the outer face of the wall 5 through which the product is visible or applied or included in said wall 5 or formed on the opposite face of the product 4 or embedded in the product 4, allow to obtain particular optical effects that give the device as a whole a particularly pleasant ornamental effect.

It should be noted in fact that the wall 5 has a refractive index that is different from the refractive index of the product 4 and that the outer surface of the wall 5, with the reliefs and the recesses of the design 6 (with particular reference to Figures 1 to 3), and with the opaque or semi-opaque or semitransparent surface of the background 9 in contrast with the smooth surface of the design 6, allow to obtain, for the various parts that compose the device, a different optical behavior that leads to a visual enhancement of the design 6.

Light, by passing through the wall 5 and the product 4 itself, is in fact partly reflected and partly refracted; this refraction, in combination with the diffusion produced by the opaque or semi-opaque or semitransparent surface of the background 9, produces color, depth and light effects that produce, for the figure 7 and the veil 8, a three-dimensional visual effect that makes them stand out from the background 9.

A similar effect, in terms of enhancement of the decorative element 6a, is achieved by forming the decorative element 6a on the face of the product 4 that lies opposite the wall 5, or by placing the decorative element inside the product 4, or by applying decorative elements to the outer and/or inner faces of the wall 5.

The product 4, with its coloring and/or transparency, cooperates decisively in achieving this effect.

It should also be noted that any gradual consumption of the product 4 can change the optical structure of the object, varying the resulting aesthetic effect, and at the same time providing information on the operational condition of the diffuser 1.

In practice it has been found that the device according to the invention fully achieves the intended aim, since the combination of the optical characteristics of the container with those of the product contained therein achieves a particular aesthetic-optical effect that gives the device an excellent decorative appearance.

In this manner, the device according to the invention, in addition to deodorizing and/or fragrancing an environment, has a pleasant decorative function.

Although the device according to the invention has been conceived in particular for diffusing deodorizers and/or fragrances, it can also be used to diffuse other substances, such as for example insecticides, sanitizers, insect repellents, etcetera.

In practice, the materials employed, so long as they are compatible with the specific purposes, as well as the contingent shapes and dimensions, may be any according to requirements and to the state of the art.

The disclosures in Italian Utility Model Application No. MI2002U000136 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. A device for diffusing a volatile active substance into the environment, comprising a container (2) which forms a compartment (3) for containing a solid or semisolid product (4) that contains the active substance, **characterized in that** said compartment (3) is delimited toward the outside of the container by at least one wall (5) made of transparent and/or semitransparent material, said product (4) being visible through said wall (5) of the container (2) and being transparent and/or semitransparent and/or colored, a decorative element (6, 6a, 10) that is visible from the outside of said container (2) being provided on said wall (5) of the container (2) and/or on a part of said product (4) that is visible through said wall (5) of the container (2).

2. The device according to claim 1, **characterized in that** said decorative element (6) is applied to the outer face and/or to the inner face of said wall (5) of the container (2).

3. The device according to claim 1, **characterized in that** said decorative element (6a) is formed in relief and/or recessed on the outer face and/or on the inner face of said wall (5) of the enclosure (3).

4. The device according to one or more of the preceding claims, **characterized in that** said decorative element (6a) is formed in relief and/or recessed on the face of said product (4) that is directed away from said wall (5) of the container(2).

5. The device according to one or more of the preceding claims, **characterized in that** said decorative element (10) is embedded in said product (4).

6. The device according to one or more of the preceding claims, **characterized in that** said decorative element (6, 6a, 10) comprises differently colored and/or transparent and/or opaque and/or polished and/or rough parts, formed on one of the two faces of said wall (5) and/or inside said wall (5).

7. The device according to one or more of the preceding claims, **characterized in that** said decorative element (6) comprises a figure (7) that has a smooth surface on a rough or opaque or semi-opaque or semitransparent background (9).

8. The device according to one or more of the preceding claims, **characterized in that** said product (4) has a different refractive index than said wall (5) of the container (2).

9. The device according to one or more of the preceding claims, **characterized in that** said product (4) is in multiple colors.

10. The device according to one or more of the preceding claims, **characterized in that** said decorative element (6a) comprises inclusions (10) that are embedded in said product (4) and are optically differentiated with respect to the remaining part of said product (4).

11. The device according to one or more of the preceding claims, **characterized in that** said product (4) adheres to the inner side of said wall (5).

12. The device according to one or more of the preceding claims, **characterized in that** said compartment (3) is open outward on its opposite side with respect to said wall (5).

13. The device according to one or more of the preceding claims, **characterized in that** the thickness of said wall (5) is substantially between 0.3 and 20 mm.

14. The device according to one or more of the preceding claims, **characterized in that** said container (2) is made of glass.

15. The device according to one or more of the preceding claims, **characterized in that** said container (2) is made of synthetic material.

16. The device according to one or more of the preceding claims, **characterized in that** said product (4) comprises a carrier and said volatile active substance.

17. The device according to one or more of the preceding claims,
**characterized in that** said active substance is a fragrance and/or a deodorizer.

18. The device according to one or more of the preceding claims, **characterized in that** said carrier is constituted by a wax or an alkaline salt of fatty acids.

19. The device according to one or more of the preceding claims, **characterized in that** said carrier is constituted by a hydrophilic or anhydrous gel.

20. The device according to one or more of the preceding claims, **characterized in that** said volatile active substance is present in said product (4) in a percentage between 5 and 90%.

21. The device according to one or more of the preceding claims, **characterized in that** said volatile active substance is present in said product (4) in a percentage between 30 and 80%.
